# EUROPEAN PATENT SPECIFICATION

(11) **EP 1 485 059 B1**
(45) Date of publication and mention of the grant of the patent: **14.02.2007**
(21) Application number: 02806891.4
(22) Date of filing: 18.03.2002
(51) Int. Cl.: A61K 9/10, A61K 47/32, A61K 47/38, A61K 31/136, A61P 17/10

(54) **TOPICAL DAPSONE FOR THE TREATMENT OF ACNE**
TOPISCHES DAPSON ZUR BEHANDLUNG VON AKNE
APPLICATION TOPIQUE DE DAPSONE POUR TRAITER L'ACNE

(30) Priority: 20.02.2002 US 81050
(43) Date of publication of application: 15.12.2004
(73) Proprietor: QLT USA, Inc., Fort Collins, CO 80525 (US)
(72) Inventor: OSBORNE, David, W., Santa Rosa, CA 95404-2028 (US)
(74) Representative: Leifert & Steffan
(86) International application number: PCT/US2002/008449
(87) International publication number: WO 2003/072071

(56) References cited:
- US-A- 4 829 058
- US-A- 5 863 560
- US-A- 6 060 085
- WOLF ET AL.: "Dapsone: Unapproved uses or indications" CLINICS IN DERMATOLOGY, vol. 18, 2000, pages 37-53, XP002337694
- J.J. RUSSELL: AM FAMILY PHYSICIAN, vol. 61, 2000, pages 357-366,
- N.L. SYKES ET AL.: DRUGS, vol. 48, no. 1, 1994, pages 59-70,

## Description

### FIELD OF THE INVENTION

The present invention relates to the field of dermatology. In particular, the invention relates to the use of dapsone for the manufacture of a dermatological composition for treating primary non-inflammatory acne or closed comedones by topical administration.

### BACKGROUND OF THE INVENTION

Sebaceous glands are associated with hair follicles and secrete an oily substance called sebum into the upper part of the follicles. These glands are found everywhere on the human skin, except for the soles and dorsa of the feet and the palms. In each gland, a common excretory duct is supplied by smaller ducts that originate in the acini of the gland. As the sebaceous cells move toward the center of the gland, lipid synthesis within the cells continues until there is a 100 to 150 fold increase in cell volume. The cells then rupture and lipid is expelled into the excretory stream of the gland, passing through the follicular canal and into the upper third of the hair follicle. On the skin of the face, the sebaceous glands are the predominant portion of the follicles and are called sebaceous follicles.

The follicular canal contains keratinous material, i.e., dead skin cells, from the wall of the canal, sebum from the sebaceous glands, and bacteria, predominately *Propionibacterium acnes.* In the follicular canal of patients with acne, these dead skin cells clump together to form a keratin plug. This altered pattern of keratinization is the primary structural change in the follicular canal that leads to an acne lesion.

When the follicular canal becomes blocked, a comedone is formed. The primary manifestation of acne is the closed comedone, which are small, circumscribed, elevated lesions of the follicle that are often without a visible central plug. Closed comedones (whiteheads) are non-inflammatory acne lesions. Open comedones (blackheads) consist of small follicular lesions having a central black keratin plug as a result of oxidation of melanin pigment. Open comedones develop from closed comedones as the orifice dilates. The open comedone is not an inflammatory lesion unless traumatized, i.e. picked at, by the patient. Comedones, either open or closed, are non-inflammatory. While the comedone is the primary lesion of acne, comedones are not unique to acne since they may be seen in other conditions such as senile comedones or trophic skin resulting from x-ray therapy.

Closed comedones are potential precursors to large inflammatory lesions. The dead skin cells of the comedone are permeated with lipid and *P. acnes,* and as the follicle dilates from the expanding mass of keratin and lipid, inflammation develops along the follicular wall. This can lead to follicular wall rupture which extrudes the entire contents of the comedone into the dermis, generating a greater inflammatory response. Inflammatory lesions can be small papules with an encircling inflammatory region or, depending on the site and extent of the rupture, a pustule or large tender nodule may form. Papules, pustules and nodules are the three clinical descriptions for inflammatory acne.

As summarized by Strauss (J. S. Strauss. (1991). "Biology of the Sebaceous Gland and Pathophysiology of Acne Vulgaris," Chapter 13 in Pathophysiology of Dermatologic Diseases, Second Edition. N. A. Sotor and H. Baden eds., McGraw-Hill, New York: pp. 195-210) there are four principles of acne therapy: 1) correct the pattern of altered keratinization within the follicle; 2) decrease sebaceous gland activity; 3) decrease the *P. acnes* population and/or decrease the generation of inflammatory substances by the bacterial population; and 4) produce non-inflammatory effects.

Topical retinoids such as tretinoin primarily function by correcting altered patterns of keratinization. Oral isotretinoin (13-cis retinoic acid) primarily functions by decreasing sebaceous gland activity. Antibiotic therapies such as oral minocycline or topical clindamycin primarily function by reducing the numbers or activity of *P. acnes.* Furthermore, steroids can be injected into acne lesions to produce an anti-inflammatory effect. However, topically applying steroids to acne results in an increase in acne lesions.

Dapsone was first synthesized in 1908 and has been used medically as an antibiotic and an anti-inflammatory. Dapsone is a bis(4-aminophenyl)sulfone also known as 4',4'-diaminodiphenyl sulfone, 4,4'-sulfonylbisbenzeneamine, 4,4'-sulfonyldianiline, and diaphenylsulfone. Dapsone has been used orally for the treatment of acne (C. M. Ross, *Br. J. Dermatol.* 73:367, (1961)) and been found to have a minimum inhibitory concentration with regard to *P. acnes* of about 1 microgram per milliliter (K. L. Godowski et al., *J. Invest. Dermatol.* 114:862 (2000)).

Dapsone analogs and related compounds have been described in U.S. Patent Nos. 4,829,058 and 4,912,112 to Seydel et al. The '058 patent discloses substituted bis(4-aminophenyl)sulfones useful for inhibiting growth of bacteria, mycobacteria, and plasmodia. Some of these compounds were also tested against dapsone for toxicity and anti-inflammatory activity (Coleman et al., *Environmental Toxicology and Pharmacology,* 2:389-395(1996)). In the '112 patent, substituted 2,4-diamino-5-benzyl pyrimidines having antimicrobial activity particularly against mycobacteria are described. Some of these compounds were also tested against dapsone for toxicity (Coleman et al., *J. Pharm. Pharmacol.,* 48:945-950 (1996)) and anti-inflammatory activity (Coleman et al., *J*. *Pharm. Pharmacol.,* 49:53-57 (1997)). The teachings of these references in combination with subsequent publications showed that these analogs and related compounds have activity similar to dapsone and would be expected to have similar treatment efficacy.

Topical dapsone formulations have been described in U.S. Patent No. 5,733,572 to Unger et al., and U.S. Patent Nos. 6,056,954; 6,056,955; 6,254,866; 6,248,324; and 6,277,399 to Fischetti et al. However, these compositions are not used to treat acne lesions.

U.S. Patent No. 6,200,964 discloses a topical silicone gel having salicylic acid, and optionally, a dermatological agent such as dapsone. In this formulation, salicylic acid is described as the anti-acne agent. Dapsone is not specifically included in this composition to treat acne.

While inflammatory acne lesions are currently treated in various ways, effective treatments for non-inflammatory acne lesions are lacking. Thus, new treatments for non-inflammatory acne are needed. In particular, a topical composition having dapsone is needed for the treatment of non-inflammatory acne.

### SUMMARY OF THE INVENTION

The present invention relates to the treatment of non-inflammatory acne by topically applying a dermatological composition that includes dapsone. The dermatological composition preferably includes a mixture of dissolved and microparticulate dapsone. Typically, dapsone is delivered using a semisolid aqueous gel, but other pharmaceutical carriers such as creams, lotions, solutions, ointments, and sprays may also be used. The dermatological composition that is topically applied may also include additives such as preservatives, antioxidants, fragrances, colorants, or sunscreens. The dermatological composition having dapsone may also be topically applied to prevent non-inflammatory acne lesions from progressing to inflammatory acne lesions.

### DETAILED DESCRIPTION OF THE INVENTION

The invention treats non-inflammatory acne, by the topical application of a dermatologic composition comprising dapsone. A topical composition including dapsone for acne treatment has been described in U.S. Patent Nos. 5,863,560, and 6,060,085 to Osborne. The composition is a combination of microparticulate and dissolved dapsone that allows optimal delivery of dapsone to the upper follicle and drug penetration to the site of inflammation. As used herein, the term "treat", "treatment", or "treating" refers to the reduction in number and/or severity of individual acne lesions, prevention of the development of acne lesions, or global improvement in the appearance of acne lesions.

The term "topical" as used herein refers to the route of administration of a dermatological composition that involves direct application to the body part being treated, e.g., the skin. Examples of topical application include application to the skin of gels or other semisolids to rub-on, solutions to spray, or liquids to be applied by an applicator. Rinse-off application with washes, cleansers, or shampoos are also examples of topical application. Typically, areas of the body suitable for application of the dermatological composition include the skin of the face, throat, neck, scalp, chest, back, ears, and other skin sites where acne lesions may occur.

*P. acnes* is a primary factor in the formation of papules, pustules, and nodules, the inflammatory lesions of acne. The antimicrobial and anti-inflammatory properties of dapsone have been well described over the nearly 100 year use of the drug, implicating dapsone as a favorable drug candidate for the treatment of inflammatory acne. The role of *P. acnes,* while not fully understood, is not considered a primary factor in the development of non-inflammatory lesions. Thus, it would be unexpected that topical dapsone would significantly reduce the number of non-inflammatory lesions. However, our experimental data demonstrate that the number of non-inflammatory acne lesions are reduced as a result of treatment with topical dapsone.

### TOPICAL DAPSONE COMPOSITIONS

By use of the term "dapsone" it is meant the chemical compound dapsone having the chemical formula C₁₂H₁₂N₂O₂S as well as bis(4-aminophenyl)sulfone, 4',4'-diaminodiphenyl sulfone and its hydrates, 4,4'-sulfonylbisbenzeneamine, 4,4'-sulfonyldianiline, and diphenylsulfone.

Non-inflammatory acne is treated by topically applying a dermatological composition comprising dapsone. Preferably, the dermatological composition is part of a novel pharmaceutical carrier system that is a semisolid aqueous gel, wherein the composition exhibits an optimal balance between dissolved dapsone that is available to cross through the stratum corneum to become systemically available, and microparticulate dapsone that is retained in or above the stratum corneum to serve as a reservoir or to provide dapsone to the supracorneum zone. The microparticulate dapsone may comprise a crystalline precipitant or an amorphous precipitant.

### Dapsone Topical Gel

Optimal balance is accomplished by having a semisolid gel carrier system in which microparticulate dapsone precipitates are formed in reproducible ratios with respect to the dissolved dapsone. For the composition to have a wide range of applicability, the microparticulate to dissolved dapsone ratio preferably should be no greater than five, at therapeutic levels of applied active dapsone.

A composition having a microparticulate to dissolved dapsone ratio of less than two may provide the greatest amount of pharmaceutical available for immediate partition out of the stratum corneum and into the viable epidermis. This should provide minimum reservoir capacity, but may not maintain sustained delivery or provide maximum activity in the supracorneum zone. A composition having a microparticulate to dissolved dapsone ratio of two or greater may have a reduced amount of drug available for immediate partition out of the stratum corneum and into the viable epidermis. This provides maximum reservoir capacity, and maintains sustained delivery, providing maximum activity in the supracorneum zone. In an example of a dermatological composition of this inventive method, the ratio for microparticulate dapsone to dissolved dapsone should be no greater than 50, preferably no greater than 10, and most preferably no greater than 5. Drug delivery from the microparticulate/dissolved dapsone formulation may be optimized to provide higher levels of drug to the supracorneum zone, while maintaining the level of drug partitioning out of the stratum corneum and into the viable epidermis, despite 10-fold increases in the amount of pharmaceutical applied to the skin.

In one embodiment, the dermatological composition that is applied comprises a semi-solid or gel-like vehicle that may include a polymer thickener, water, preservatives, active surfactants or emulsifiers, antioxidants, sunscreens, and a solvent or mixed solvent system. The solvent or mixed solvent system is important to the formation of the microparticulate to dissolved dapsone ratio. The formation of the microparticulate, however, should not interfere with the ability of the polymer thickener or preservative systems to perform their functions.

Polymer thickeners that may be used include those known to one skilled in the art, such as hydrophilic and hydroalcoholic gelling agents frequently used in the cosmetic and pharmaceutical industries. Preferably, the hydrophilic or hydroalcoholic gelling agent comprises "CARBOPOL®" (B.F. Goodrich, Cleveland, OH), "HYPAN®" (Kingston Technologies, Dayton, NJ), "NATROSOL®" (Aqualon, Wilmington, DE), "KLUCEL®" (Aqualon, Wilmington, DE), or "STABILEZE®" (ISP Technologies, Wayne, NJ). Preferably, the gelling agent comprises between about 0.2% to about 4% by weight of the composition. More particularly, the preferred compositional weight percent range for "CARBOPOL®" is between about 0.5% to about 2%, while the preferred weight percent range for "NATROSOL® and "KLUCEL®" is between about 0.5% to about 4%. The preferred compositional weight percent range for both "HYPAN®" and "STABILEZE®" is between about 0.5% to about 4%.

"CARBOPOL®" is one of numerous cross-linked acrylic acid polymers that are given the general adopted name carbomer. These polymers dissolve in water and form a clear or slightly hazy gel upon neutralization with a caustic material such as sodium hydroxide, potassium hydroxide, triethanolamine, or other amine bases. "KLUCEL®" is a cellulose polymer that is dispersed in water and forms a uniform gel upon complete hydration. Other preferred gelling polymers include hydroxyethylcellulose, hydroxypropylcellulose, cellulose gum, MVA/MA copolymers, MVE/MA decadiene crosspolymer, PVM/MA copolymer, or a combination thereof. Preservatives may also be used in this dermatological composition and preferably comprise about 0.05% to 0.5% by weight of the total composition. The use of preservatives assures that if the product is microbially contaminated, the formulation will prevent or diminish microorganism growth. Some preservatives useful in this invention include methylparaben, propylparaben, butylparaben, chloroxylenol, sodium benzoate, DMDM Hydantoin, 3-Iodo-2-Propylbutyl carbamate, potassium sorbate, chlorhexidine digluconate, or a combination thereof.

Titanium dioxide may be used as a sunscreen to serve as prophylaxis against photosensitization. Alternative sunscreens include methyl cinnamate. Moreover, BHA may be used as an antioxidant, as well as to protect ethoxydiglycol and/or dapsone from discoloration due to oxidation. An alternate antioxidant is BHT.

In one embodiment, the dermatological composition that is applied includes 0.5% to 4.0% carbomer and 0.5% to 10% dapsone that exists in both a dissolved state and a microparticulate state. In another embodiment, the dermatological composition comprises 1% carbomer, 80-90% water, 10% ethoxydiglycol, 0.2% methylparaben, and 0.3% to 3.0% dapsone including both microparticulate dapsone and dissolved dapsone, and 2% caustic material. More particularly, the carbomer may include "CARBOPOL® 980" and the caustic material may include sodium hydroxide solution.

In a preferred embodiment, the composition comprises dapsone and ethoxydiglycol, which allows for an optimized ratio of microparticulate drug to dissolved drug. This ratio determines the amount of drug delivered, compared to the amount of drug retained in or above the stratum corneum to function in the supracorneum domain. The system of dapsone and ethoxydiglycol may include purified water combined with "CARBOPOL®" gelling polymer, methylparaben, propylparaben, titanium dioxide, BHA, and a caustic material to neutralize the "CARBOPOL®."

### Dapsone Topical Cream or Lotion

In another embodiment, dapsone may be applied as a topical cream or lotion in which dapsone is dissolved or dispersed or both partially dissolved and partially dispersed. Topical creams or lotions may be either oil-in-water emulsions or water-in-oil emulsions. The oil phase may include but is not limited to fatty alcohols, acids, or esters such as cetyl palmitate, cetyl alcohol, stearyl alcohol, stearic acid, isopropyl stearate, glycerol stearate, mineral oil, white petrolatum, or other oils alone or in combination.

Emulsifiers that may be added to the composition include, but are not limited to, steareth 20, ceteth 20, sorbitan sesquioleate, sorbitan mono-oleate, propylene ,glycol stearate, dosium lauroyl sarcosinate, polysorbate 60, or combination. Preservatives, antioxidants, fragrances, colorants, sunscreens, thickeners, and other additives required to achieve pharmaceutical or cosmetically acceptable or preferred product may also be included. However, topical creams and lotions are not limited to these components since one skilled in the art will be aware of additional components useful in the formulation of topical creams and lotions.

### Dapsone Topical Solution or Suspension

In another embodiment dapsone may be applied as a solution or suspension. These are fluid solvent or mixed-solvent systems including, water, ethanol, propylene glycol, glycerol, polyethylene glycol, ethyl acetate, propylene carbonate, n-methyl pyrolidone, triethanolamine, 1, 4-butanediol, triacetin, diacetin, dimethyl isosorbide alone or in combination. Preservatives, antioxidants, fragrances, colorants, sunscreens, thickeners, suspending agents, enhancers, and other additives required to achieve pharmaceutically or cosmetically acceptable or preferred product may also be included. Again, topical solutions or suspensions are not limited to these components, since one skilled in the art will be aware of additional components useful in the formulation of topical solutions or suspensions.

### Other Dapsone Topical Formulations

Dapsone may also be applied using a pharmaceutical or cosmetic carrier form such as an ointment, roll-on or stick product, micro-emulsion, shake powder, an aerosolized spray or mousse, a pump spray or mousse, or bath additive. Examples of ointments include essentially non-aqueous mixtures of petrolatum, lanolin, polyethylene glycol, plant or animal oils, either hydrogenated or otherwise chemically modified. An ointment may also contain a solvent in which dapsone is either fully or partially dissolved. Additional pharmaceutical carriers will be known to those skilled in the art.

### METHOD FOR PREPARING THE DAPSONE DERMATOLOGICAL COMPOSITION

The present invention also provides methods for preparing the dermatological compositions described above. In a general form, the method for producing a dermatological gel composition having dissolved dapsone and microparticulate dapsone precipitates comprises the steps of completely dissolving dapsone in a solvent or solvent mixture; adding and adequately dispersing a polymeric thickener in water; and combining the dissolved dapsone with the dispersed polymeric thickener. Alternatively, water may be slowly added to the dissolved dapsone, followed by the addition of a polymeric thickener. Ethoxydigylcol and 1-methyl-2-pyrollidone are preferred solvents for use in the topically applied dermatological composition.

In one preferred embodiment, the method for preparing a topically applied dermatological composition having dissolved and microparticulate dapsone comprises the steps of forming a homogenous dispersion by stirring purified water vigorously enough to form a vortex and sifting gel polymer into the vortex formed in the water while continuing to stir; forming a pharmaceutical component by dissolving methyl paraben and propylparaben in ethoxydiglycol by mixing to form a solution, and mixing dapsone with the solution until the pharmaceutical is dissolved; mixing the pharmaceutical component with the homogenous dispersion to form a microparticulate dapsone dispersion; and adding a caustic material.

The order in which reagents are combined may be important, depending on the particular reagents necessary for the target mixture. For example, after a pharmaceutical such as dapsone is dissolved in a solvent such as ethoxydiglycol, water may be slowly added to the dapsone in the ethoxydiglycol solution, or the dapsone in ethoxydiglycol solution may be added to the water with mixing. Adding the dapsone in ethoxydiglycol solution to water may result in less polydispersity in the size of the microparticulates than adding water to the dapsone in ethoxydiglycol solutions. The carbomer is generally dispersed in the water component of the formulation, while the remaining ingredients will be dissolved or dispersed in whichever of the two components are best for dissolving or dispersing the ingredient. For example, it is suggested to dissolve methylparaben, propylparaben, and BHA in ethoxydiglycol. After the ethoxydiglycol component and water component are combined, neutralizer is added to formulate the gel.

### SPECIFIC APPLICATIONS OF TOPICAL DAPSONE

The invention relates to the use of dapsone for the manufacture of a dermatological composition for reducing the number of non-inflammatory acne lesions by topical administration. Furthermore, in another embodiment, a use is provided for topically applying a dermatological composition having dapsone to prevent closed comedones (non-inflammatory acne) from becoming inflamed papules, pustules, or nodules. However, if the follicular canal ruptures, dapsone would also help to reduce the resultant inflammation. Typically, the dermatological composition having dapsone is applied once daily, but may be applied more frequently if desired.

### EXAMPLES

The following examples are provided to show that topically applied dapsone has unexpected therapeutic benefit in the treatment of non-inflammatory acne lesions. Dapsone was expected to reduce the degree of inflammation and the number of inflammatory lesions. The known mechanisms of dapsone activity did not anticipate an improvement in the number of non-inflammatory lesions.

### EXAMPLE 1

A 20-year-old white male applied 1% dapsone topical gel once daily as part of an open label, pharmacokinetic clinical study. This patient had 115 non-inflammatory lesions (closed comedones) at baseline. The number of non-inflammatory lesions decreased to 103 after seven days of treatment, 31 non-inflammatory lesions after 24 days, and 13 non-inflammatory lesions after 21 days of treatment with 1% dapsone topical gel applied once daily.

### EXAMPLE 2

A four week, open label, dose-ranging study was completed in 18 - 39-year-old patients having mild to moderate acne. The number of pustules, papules, comedones, and nodules were counted at baseline and after 28 days of topical dapsone therapy for each patient. The number of papules, pustules, and nodules were combined to give the total number of inflammatory lesions while the number of comedones provided the total non-inflammatory lesion count. Four males and seven females applied 1% dapsone topical gel once daily; five males and seven females applied 1% dapsone topical gel twice daily; seven males and six females applied 5% dapsone topical gel once daily; and four males and eight females applied 5% dapsone topical gel twice daily. The average percent lesion reduction for each of the four dosage groups after 28 days of treatment is shown in Table 1. These results can be compared to a 0.1 % Tretinoin cream. (FOI Services, Inc., Gaithersburg, MD, releasable documents for ANDA 75-213). This maximum strength topical retinoid produced a 26% (test article) to 27% (reference article) reduction in non-inflammatory lesions after four weeks of treatment.

**TABLE 1**

| | | Percent Lesion Reduction | |
|---|---|---|---|
| | | Inflammatory | Non-Inflammatory |
| 1% Dapsone | Once Daily Dosing | 13% | 53% |
| 1 % Dapsone | Twice Daily Dosing | 53% | 45% |
| 5% Dapsone | Once Daily Dosing | 48% | 32% |
| 5% Dapsone | Twice Daily Dosing | 47% | 39% |

### EXAMPLE 3

Table 2 shows the results from a multicenter, double-blind, randomized, parallel-design study in which patients were randomized to receive either 3 % dapsone topical gel, 5% dapsone topical gel, or vehicle control once daily for 12 weeks. Inflammatory lesion counts include data from Portland, Cheery Creek, Orange County, and Denver Center clinical sites, with the number of patients being 20, 15, and 18 for the 5% dapsone, 3% dapsone, and vehicle control groups respectively. Patients were male or female aged 13-years or older. This was the first study in which a vehicle control was used. Thus, any reduction in non-inflammatory lesions beyond about 13% is due to the effect of topical dapsone.

**TABLE 2**

| | | Percent Lesion Reduction | |
|---|---|---|---|
| | | Inflammatory | Non-Inflammatory |
| Vehicle | Once Daily Dosing | 28% | 13% |
| 3% Dapsone | Once Daily Dosing | 46% | 37% |
| 5% Dapsone | Once Daily Dosing | 45% | 28% |

## Claims

1. Use of dapsone for the manufacture of a dermatological composition to be topically applied for reducing a number of non-inflammatory acne lesions.

2. The use of claim 1 wherein said dermatological composition comprises dissolved dapsone and microparticulate dapsone.

3. The use of claim 2 wherein said dermatological composition is a semisolid aqueous gel.

4. The use of claim 2 wherein said dermatological composition is a cream.

5. The use of claim 2 wherein said dermatological composition is a lotion.

6. The use of claim 2 wherein said dermatological composition is a suspension.

7. The use of claim 2 wherein said dermatological composition is an ointment.

8. The use of claim 2 wherein said dermatological composition is a spray.

9. The use of claim 2 wherein said dermatological composition further comprises an additive selected from the group consisting of a preservative, an antioxidant, a fragrance, a colorant, and a sunscreen.

10. The use of claim 1 wherein said dermatological composition is a semisolid aqueous gel.

11. The use of claim 1 wherein said dermatological composition is a cream.

12. The use of claim 1 wherein said dermatological composition is a lotion.

13. The use of claim 1 wherein said dermatological composition is a solution.

14. The use of claim 1 wherein said dermatological composition is an ointment.

15. The use of claim 1 wherein said dermatological compositions is spray.

16. The use of claim 1 wherein said dermatological composition further comprises an additive selected from the group consisting of a preservative, an antioxidant, a fragrance, a colorant, and a sunscreen.

17. The use of claim 1 wherein said dermatological compositions comprises 3 % dapsone.

18. The use of claim 1 wherein said dermatological composition comprises 5 % dapsone.

19. The use of dapsone for the manufacture of a dermatological composition to be applied topically for preventing a non-inflammatory acne lesion from becoming an inflammatory acne lesion.

## Patentansprüche

1. Verwendung von Dapson für die Herstellung einer dermatologischen Zusammensetzung, die topisch zur Verringerung einer Reihe von nicht inflammatorischen Akneläsionen anzuwenden ist.

2. Verwendung nach Anspruch 1, wobei die dermatologische Zusammensetzung gelöstes Dapson und Dapson in Form von Mikropartikeln umfasst.

3. Verwendung nach Anspruch 2, wobei die dermatologische Zusammensetzung ein halbfestes wässriges Gel ist.

4. Verwendung nach Anspruch 2, wobei die dermatologische Zusammensetzung eine Creme ist.

5. Verwendung nach Anspruch 2, wobei die dermatologische Zusammensetzung eine Lotion ist.

6. Verwendung nach Anspruch 2, wobei die dermatologische Zusammensetzung eine Suspension ist.

7. Verwendung nach Anspruch 2, wobei die dermatologische Zusammensetzung eine Salbe ist.

8. Verwendung nach Anspruch 2, wobei die dermatologische Zusammensetzung ein Spray ist.

9. Verwendung nach Anspruch 2, wobei die dermatologische Zusammensetzung des Weiteren ein Additiv umfasst, das ausgewählt ist aus der Gruppe bestehend aus einem Konservierungsmittel, einem Antioxidans, einem Duftstoff, einem Farbstoff und einem Sonnenschutzmittel.

10. Verwendung nach Anspruch 1, wobei die dermatologische Zusammensetzung ein halbfestes wässriges Gel ist.

11. Verwendung nach Anspruch 1, wobei die dermatologische Zusammensetzung eine Creme ist.

12. Verwendung nach Anspruch 1, wobei die dermatologische Zusammensetzung eine Lotion ist.

13. Verwendung nach Anspruch 1, wobei die dermatologische Zusammensetzung eine Lösung ist.

14. Verwendung nach Anspruch 1, wobei die dermatologische Zusammensetzung eine Salbe ist.

15. Verwendung nach Anspruch 1, wobei die dermatologische Zusammensetzung ein Spray ist.

16. Verwendung nach Anspruch 1, wobei die dermatologische Zusammensetzung des Weiteren ein Additiv umfasst, das ausgewählt ist aus der Gruppe bestehend aus einem Konservierungsmittel, einem Antioxidans, einem Duftstoff, einem Farbstoff und einem Sonnenschutzmittel.

17. Verwendung nach Anspruch 1, wobei die dermatologische Zusammensetzung 3 % Dapson umfasst.

18. Verwendung nach Anspruch 1, wobei die dermatologische Zusammensetzung 5 % Dapson umfasst.

19. Verwendung von Dapson für die Herstellung einer dermatologischen Zusammensetzung, die topisch anzuwenden ist, um zu verhindern, dass eine nicht inflammatorische Akneläsion sich zu einer inflammatorischen Akneläsion entwickelt.

## Revendications

1. Utilisation de dapsone pour la fabrication d'une composition dermatologique pour application topique pour réduire un certain nombre de lésions acnéiques non inflammatoires.

2. Utilisation selon la revendication 1, dans laquelle ladite composition dermatologique comprend de la dapsone dissoute et de la dapsone microparticulaire.

3. Utilisation selon la revendication 2, dans laquelle ladite composition dermatologique est un gel aqueux semi-solide.

4. Utilisation selon la revendication 2, dans laquelle ladite composition dermatologique est une crème.

5. Utilisation selon la revendication 2, dans laquelle ladite composition dermatologique est une lotion.

6. Utilisation selon la revendication 2, dans laquelle ladite composition dermatologique est une suspension.

7. Utilisation selon la revendication 2, dans laquelle ladite composition dermatologique est un onguent.

8. Utilisation selon la revendication 2, dans laquelle ladite composition dermatologique est un pulvérisateur.

9. Utilisation selon la revendication 2, dans laquelle ladite composition dermatologique comprend également un additif choisi dans le groupe constitué par un conservateur, un antioxydant, un parfum, un colorant, et un écran solaire.

10. Utilisation selon la revendication 1, dans laquelle ladite composition dermatologique est un gel aqueux semi-solide.

11. Utilisation selon la revendication 1, dans laquelle ladite composition dermatologique est une crème.

12. Utilisation selon la revendication 1, dans laquelle ladite composition dermatologique est une lotion.

13. Utilisation selon la revendication 1, dans laquelle ladite composition dermatologique est une solution.

14. Utilisation selon la revendication 1, dans laquelle ladite composition dermatologique est un onguent.

15. Utilisation selon la revendication 1, dans laquelle ladite composition dermatologique est un pulvérisateur.

16. Utilisation selon la revendication 1, dans laquelle ladite composition dermatologique comprend également un additif choisi dans le groupe constitué par un conservateur, un antioxydant, un parfum, un colorant, et un écran solaire.

17. Utilisation selon la revendication 1, dans laquelle ladite composition dermatologique comprend 3 % de dapsone.

18. Utilisation selon la revendication 1, dans laquelle ladite composition dermatologique comprend 5 % de dapsone.

19. Utilisation de dapsone pour la fabrication d'une composition dermatologique pour application topique pour empêcher l'évolution d'une lésion acnéique non inflammatoire en lésion acnéique inflammatoire.
